(19) Europäisches Patentamt
European Patent Office
Office européen des brevets

(11) **EP 3 069 659 A1**

(12) **EUROPEAN PATENT APPLICATION**
published in accordance with Art. 153(4) EPC

(43) Date of publication:
**21.09.2016 Bulletin 2016/38**

(51) Int Cl.:
*A61B 6/02* *(2006.01)*     *G01N 23/04* *(2006.01)*

(21) Application number: **14859707.3**

(86) International application number:
**PCT/CN2014/071096**

(22) Date of filing: **22.01.2014**

(87) International publication number:
**WO 2015/066977 (14.05.2015 Gazette 2015/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.11.2013   CN 201310557196**

(71) Applicant: **University of Science and Technology of China**
**Anhui 230026 (CN)**

(72) Inventors:
• **LU, Yalin**
**Hefei**
**Anhui 230026 (CN)**

• **TIAN, Yangchao**
**Hefei**
**Anhui 230026 (CN)**
• **GAO, Kun**
**Hefei**
**Anhui 230026 (CN)**
• **LIU, Gang**
**Hefei**
**Anhui 230026 (CN)**
• **WANG, Zhili**
**Hefei**
**Anhui 230026 (CN)**

(74) Representative: **Graf von Stosch, Andreas et al**
**Graf von Stosch**
**Patentanwaltsgesellschaft mbH**
**Prinzregentenstraße 22**
**80538 München (DE)**

(54) **APPARATUS AND METHOD FOR X-RAY GRATING PHASE-CONTRAST IMAGING**

(57)     A hard X-ray grating phase-contrast imaging apparatus with large field-of-view, high contrast and low dose and the method thereof. The apparatus includes a source emitter (31), a source grating (G0), a beam splitting grating (G1), an analyzer grating (G2) and a detector (32) arranged in sequence on a transmission path of the source emitter (31). The beam splitting grating (G1) has a period of 30 to 50 $\mu$m and a depth to width ratio not greater than 20. The apparatus can provide a high image contrast and a low radiation dose and realize a phase-contrast imaging with high energy and a large field-of-view by increasing the grating period, increasing a duty cycle of the beam splitting grating while increasing the distance between an object and the analyzer grating. The apparatus can also utilize the conventional polychromatic X-ray sources and available process for manufacturing gratings, and be suitable for clinical use.

Fig. 3

**Description**

**BACKGROUND OF THE INVENTION**

**Field of the Invention**

The present disclosure relates to technical fields of such as medical imaging, non-destructive examination and public security inspection, in particular, to an apparatus and a method for X-ray grating phase-contrast imaging, for example, an apparatus and a method for hard X-ray grating phase-contrast imaging with large field- of-view, high contrast and low dose.

**Description of the Related Art**

In the conventional X-ray imaging technology, an image contrast is derived from difference in absorption property of an object to X-ray. Thus, when such technology is used to image an object composed of heavy elements such as metals, and bones, a very high image contrast may be achieved. However, when the object (for example human tissue, organic polymer materials) mainly composed of light elements such as carbon, hydrogen, and oxygen, the image contrast becomes too low to obtain useful information of the object.

In contrast to the traditional absorption contrast imaging, X-ray phase-contrast imaging technology is able to achieve a very high image contrast when it images the object mainly composed of light elements such as carbon, hydrogen, and oxygen. In hard X-ray wave band (10 to 100keV), for elements such as carbon, hydrogen, and oxygen, its refractivity phase term is over 1000 times of its absorption term (see Momose A, Fukuda J, "Phase contrast radiographs of nonstained rat cerebellar specimen", Med. Phys. 22, 375 (1995)). Thus, for weak absorption matters such soft tissues, measuring phase shift information upon the X-ray passing through the object may be much more efficient than detecting amplitude attenuation information. The X-ray phase-contrast imaging technology is just the imaging technology that forms an image contrast by recording variation of phase (i.e., phase shift) of the X-ray through the object. In comparison with the traditional absorption contrast imaging, the phase-contrast imaging is able to achieve higher image contrast and lower radiation dose. Since the mid-1990s, as the theories and experiment methods develop, the X-ray phase-contrast imaging technology has been widely applied in research and developments in various fields such as medical science, biology, and material science.

At present, a hard X-ray phase-contrast imaging method mainly includes four types: crystal interferometer imaging (see Bonse U, Hart M,"An X-ray interferometer", Appl. Phys. Lett. 6, 155 (1965) and Momose A, Takeda T, Itai Y, "Phase-contrast X-ray computed tomography for observing biological soft tissues", Nat. Med. 2, 473 (1996)), grating phase contrast imaging (see Momose A, Kawamoto S, Koyama I, Hamaishi Y, Takai K, Suzuki Y, "Demonstration of X-ray Talbot interferometry", Jpn. J. Appl. Phys. 42(7B), L866 (2003) and Pfeiffer F, Weitkamp T, Bunk O et al., "Phase retrieval and differential phase-contrast imaging with low-brilliance X-ray sources", Nat. Phys. 2, 258 (2006)), diffraction enhanced imaging (see Davis T J, Gao D, Gureyev T E et al., "Phase-contrast imaging of weakly absorbing materials using hard X-rays", Nature 373, 595 (1995) and Chapman D, Thomlinson W, Johnston RE et al., "Diffraction enhanced x-ray imaging", Phys. Med. Biol. 42, 2015 (1997)) and phase propagation imaging ( see Snigirev A, Snigireva I, Kohn V, Kuznetsov S, Schelokov I et al., "On the possibilities of x-ray phase contrast microimaging by coherent high-energy synchrotron radiation", Rev. Sci. Instrum. 66, 5486 (1995) and Wilkins S W, Gureyev T E, Gao D et al., "Phase-contrast imaging using polychromatic hard X-rays", Nature 384, 335 (1996)).

In the above types, the crystal interferometer imaging needs a synchrotron radiation source which has imaging field-of-view of only a few centimeters and thus needs a very high stability of an imaging apparatus (in order of wavelength of an incident X-ray). Therefore, it is not practical to be used in clinical medicine imaging. The diffraction enhanced imaging method needs very high monochromaticity and collimation of an illumination X-ray and is also mainly achieved by the synchrotron radiation source, which has imaging field-of-view of only a few centimeters limited by size of crystal and thus fails to meet the requirements for the field such as clinical medical diagnosis. The phase propagation imaging method needs very high spatial coherence of the illumination X-ray and fails to use conventional X-ray sources efficiently; in addition, its imaging field-of-view also has only a few centimeters and thus it cannot be used in the field such as clinical medical imaging.

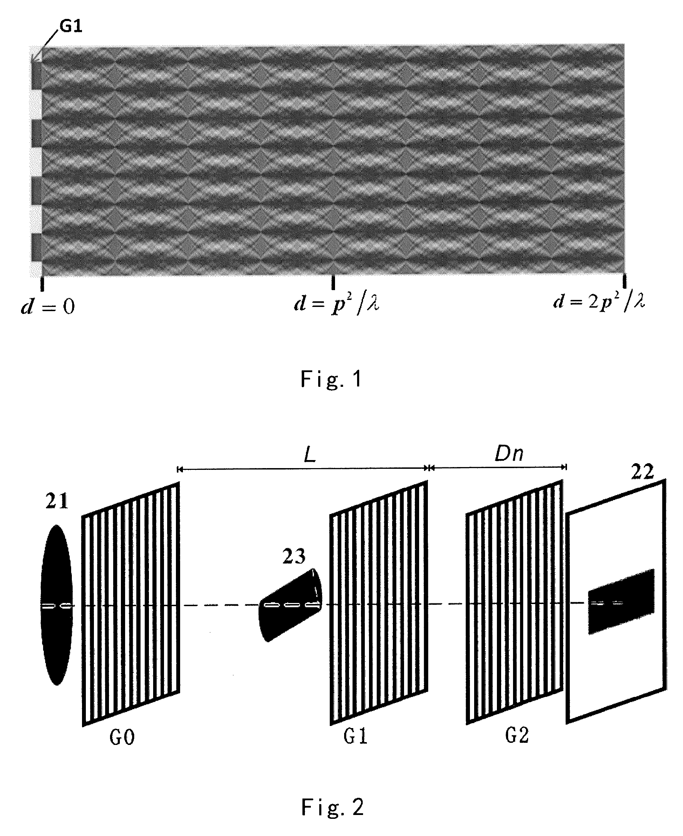

In 2006, Talbot-Lau interferometer was proposed, which provides a chance of using the hard X-ray phase-contrast imaging in clinical application. The imaging principle of the Talbot-Lau interferometer is based on fraction Talbot self imaging effects of the X-ray phase grating. As illustrated in Fig. 1, with illumination by spatial partially coherent X-ray, the diffraction intensity distribution of the X-ray will vary periodically in a lateral direction at a special distance from the phase grating G1 and behind the phase grating G1. Such phenomenon is called as fraction Talbot self imaging effect and the distance is called as fraction Talbot distance. The spatial partially coherent illumination needs the period of the phase grating G1 is not greater than a spatial coherent length of the illumination X-ray.

The Talbot-Lau interferometer shown in Fig. 2 is composed of an X-ray source 21, a source grating G0, a phase grating G1, an absorption grating G2 and a detector 22. The phase grating G1 has a phase shift equal to $\pi$, and a period *pl* less than the spatial coherent length *Ls* of the illumination X-ray:

$$Ls = \lambda L/s$$

where $\lambda$ is a wavelength of the illumination X-ray, $\lambda \leq 0.1$ nanometer and *s* is size of the illumination X-ray source. In the Talbot-Lau interferometer, the spatial coherent length *Ls* has only a few micrometers, and thus the period *pl* of the phase grating G1 is only a few micrometers; the period *p2* of the absorption grating G2 is equal to the self imaging period of the phase grating G1 and thus is only a few micrometers; the distance between G1 and G2 is equal to a certain order Talbot-Lau distance *Dn* of the phase grating G1,

$$Dn = \frac{L * n\left(p1\right)^2 \big/ 8\lambda}{L - n\left(p1\right)^2 \big/ 8\lambda}$$

where n is a positive odd number such as 1, 3, 5, called as a Talbot order.

The Talbot-Lau interferometer has a weak requirement on the monochromaticity of an illumination X-ray and may cooperate with a conventional polychromatic X-ray source to achieve a phase-contrast imaging. However, the Talbot-Lau interferometer has limitations in principle and method and thus cannot achieve high energy and large field-of-view for imaging such that it fails to be used in the field such as clinical medical diagnosis.

On one hand, the Talbot-Lau interferometer cannot achieve high energy (>60keV) imaging. As discussed above, the Talbot-Lau interferometer is based on fraction Talbot self imaging principle. It needs spatial partially coherent illumination, thus the period of the grating (G1 and G2) is limited to the spatial coherent length of the illumination X-ray, which has only a few micrometers (typically 2 to 8 micrometers in experiments). In applications such as clinical medical diagnosis, it needs to image thick samples (tens of centimeters) such as animals, human body, which requires the illumination X-ray to have sufficiently high energy in order to ensure relative high transmissivity. For example, when the energy of the illumination X-ray is equal to 30keV, the penetration depth of the X-ray in water has only three centimeters; when the energy of the illumination X-ray is enhanced to 60keV, the penetration depth of the X-ray in water will be increased to 34 centimeters. Thus, the clinical medical diagnosis requires the illumination X-ray to have energy not less than 60keV, so as to maintain sufficiently high transmissivity of X-ray. In such high energy range, the height of strong absorption material such as aurum, lead in the absorption grating G2 is required to be not less than hundreds of micrometers to achieve absorptivity more than 90% for X-ray. Taking aurum as an example, when the illumination X-ray has energy of 80keV, it needs scribe lines to have a depth not less than 545 micrometers. It means that the grating has a depth to width ratio (the ratio of the depth to the width of a scribe line) greater than several tens, even equal to several hundreds. The conventional micro-manufacturing technology cannot meet such requirements of design. It is the bottleneck that cannot be removed for the Talbot-Lau interferometer imaging apparatus in applications such as clinical medical diagnosis.

Further, the Talbot-Lau interferometer requires use of grating with a period in micrometers. At present, it has to image the object with size of tens of millimeters, but fails to achieve a large field-of-view (>100$\times$100mm$^2$) for imaging. In summary, the Talbot-Lau interferometer is still limited to research in labs now, and fails to be applied in practical fields such as clinical medical imaging.

## SUMMARY

The technical problem to be solved in the present application is to remove the bottleneck that the conventional X-ray grating phase-contrast imaging apparatus and method fail to achieve high energy (>60keV) and large field-of-view (>100$\times$100mm$^2$) for imaging such that it fails to be applied in technical field such as clinical medical diagnosis.

In order to solve the above technical problem, the present application provides an apparatus for X-ray grating phase-contrast imaging, including: a source emitter, a source grating, a beam splitting grating, an analyzer grating and a detector arranged in sequence on a transmission path of the source emitter, wherein the beam splitting grating has a period of 30 to 50$\mu$m and a depth to width ratio not greater than 20.

In an embodiment, the source emitter (31) is a hard X-ray source.

In an embodiment, the hard X-ray source has energy greater than 60keV.

In an embodiment, the source grating (G0), the beam splitting grating (G1) and the analyzer grating (G2) are all absorption gratings.

In an embodiment, the beam splitting grating (G1) has a duty cycle less than 0.5.

In an embodiment, the beam splitting grating (G1) has a size greater than 100mm × 100mm.

In an embodiment, there is a distance of 1 to 2 meters between the beam splitting grating (G1) and the analyzer grating (G2).

In an embodiment, the source grating, the beam splitting grating and the analyzer grating(G0, G1, G3) are all curved surface gratings and meet normal incidence condition for an incident ray in an imaging field-of-view range all along.

In an embodiment, the beam splitting grating (G1) and the analyzer grating (G2) are both two dimensional gratings.

In an embodiment, the source emitter (31) is a neutron emitter.

In addition, an embodiment of the present invention also provides an X-ray grating phase-contrast imaging method, including: arranging a source grating, a beam splitting grating, an analyzer grating and a detector in sequence on a transmission path of a source emitter; putting an object against the beam splitting grating and towards the analyzer grating; splitting a beam source from the source emitter into a plurality of separate beam sources by the source grating and generating an intensity array in a plane in which the analyzer grating is located by the beam splitting grating, wherein the object refracts a ray from the beam source to cause a lateral movement of the intensity array, which is detected by the analyzer grating and converted into an intensity variation which is recordable by the detector, and wherein the beam splitting grating (G1) has a period of 30 to 50$\mu$m and a depth to width ratio not greater than 20.

In an embodiment of the present invention, the source emitter (31) is a hard X-ray source.

In an embodiment of the present invention, the hard X-ray source has energy greater than 60keV.

In an embodiment of the present invention, the source grating (G0), the beam splitting grating (G1) and the analyzer grating (G2) are all absorption gratings.

In an embodiment of the present invention, the beam splitting grating (G1) has a duty cycle less than 0.5.

In an embodiment of the present invention, the beam splitting grating (G1) has a duty cycle between 0.2 and 0.4.

In an embodiment of the present invention, the beam splitting grating (G1) has a size greater than 100mm×100mm.

In an embodiment of the present invention, there is a distance of 1 to 2 meters between the beam splitting grating (G1) and the analyzer grating (G2).

In an embodiment of the present invention, the source grating, the beam splitting grating and the analyzer gratin (G0, G1, G3) are all curved surface gratings and meet normal incidence condition for the incident ray in an imaging field-of-view range all along.

In an embodiment of the present invention, the beam splitting grating (G1) and the analyzer grating (G2) are both two dimensional gratings.

In an embodiment of the present invention, the source emitter (31) is a neutron emitter.

By means of increasing period of the grating, optimizing the duty cycle of beam splitting grating and the distance between the object and the analyzer grating, the present application provides a phase-contrast imaging apparatus and a phase-contrast imaging method with high image contrast, low radiation dose, large field-of-view. And the present application may use the conventional polychromatic X-ray source and the conventional grating manufacturing process and is suitable for practical clinical applications. In particular, the present application has advantageous effects as described by the following five aspects:

1. it avoids the requirements to spatial coherent and monochromatic illumination and may use the conventional polychromatic X-ray source;
2. it has small depth to width ratio (5 to 20) of the grating, which is satisfied by the conventional process for manufacturing gratings;
3. it has high image contrast which is 5 to 10 times of the conventional absorption contrast;
4. it has low radiation dose which is 10% to 20% of the radiation dose of the conventional absorption contrast imaging;
5. it may achieve a phase-contrast imaging with high energy and large field-of-view.

## BRIEF DESCRIPTION OF THE DRAWINGS

Fig. 1 is a schematic view showing a fraction Talbot self imaging effects in the prior art;
Fig. 2 is a schematic view showing an X-ray Talbot-Lau interferometer in the prior art;
Fig. 3 is a schematic view showing an apparatus for X-ray grating phase-contrast imaging according to a first embodiment and a second embodiment of the present invention;

Fig. 4 is a schematic view showing an apparatus for X-ray grating phase-contrast imaging according to a third embodiment of the present invention;

Fig. 5 is a graph of image contrast of model sample v.s. a duty cycle of a grating;

Figs. 6A to 6D show results in an experiment carried out according to an embodiment of the present invention, where Fig. 6A shows the conventional absorption contrast image of a PMMA column in an air environment, Fig. 6B shows a phase-contrast image in case that the grating has a duty cycle of 0.2, Fig. 6C shows a phase-contrast image in case that the grating has a duty cycle of 0.5, and Fig. 6D shows comparisons of cross sectional profiles of the images for the above three different objects; and

Figs. 7A to 7B show results in an experiment carried out according to another embodiment of the present invention, where Fig. 7A shows a refraction image of a PMMA column and a POM column in an air environment, and Fig. 7B shows a cross sectional profile of the refraction image of a sample.

## DETAINED DESCRIPTION OF PREFERRED EMBODIMENTS OF THE INVENTION

In order to make clearer understanding of the above objects, features and advantages of the present disclosure, the present application will be described hereinafter in detail with reference to exemplary embodiments and attached drawings.

By means of improving the duty cycle of the beam splitting grating while increasing the distance between the object and the analyzer grating, the present application proposes an apparatus for X-ray grating phase-contrast imaging and a method for X-ray grating phase-contrast imaging using a large period absorption grating.

In accordance with an aspect of the present disclosure, by means of improving the duty cycle of the beam splitting grating while increasing the distance between the object and the analyzer grating, high image contrast may be obtained. By using the large period absorption grating, a large area absorption grating may be manufactured by micro-manufacturing technology, so as to achieve X-ray grating phase-contrast imaging with large field-of-view and high energy.

In accordance with another aspect of the present disclosure, by means of improving the duty cycle of the beam splitting grating while increasing the distance between the object and the analyzer grating, the radiation dose may be reduced without reducing the image contrast.

In accordance with a third aspect of the present disclosure, the apparatus and method for X-ray grating phase-contrast imaging may be applied in two dimensional imaging.

In accordance with a fourth aspect of the present disclosure, the apparatus and method for X-ray grating phase-contrast imaging may be applied in the neutron grating phase-contrast imaging.

The exemplified embodiments of the present invention will be described below.

### First Embodiment

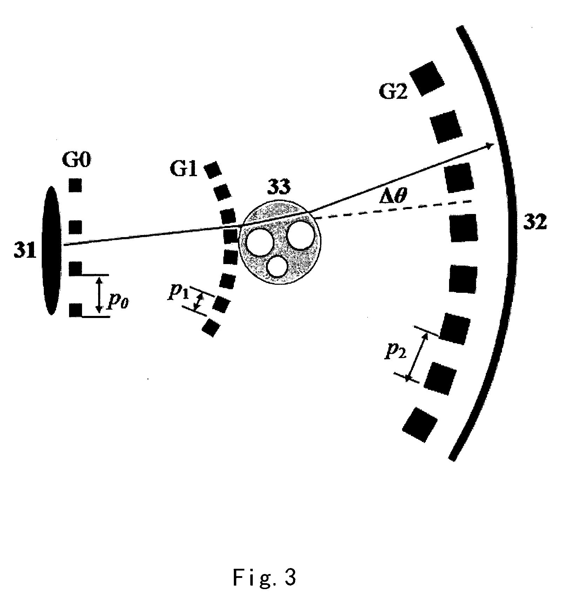

Fig. 3 is a schematic view showing an apparatus for X-ray grating phase-contrast imaging according to a first embodiment and a second embodiment of the present invention. As illustrated, the apparatus for X-ray grating phase-contrast imaging includes: an X-ray source 31, a source grating G0, a beam splitting grating G1, an analyzer grating G2 and a detector 32. The source grating G0, the beam splitting grating G1, the analyzer grating G2 and the detector 32 are arranged in sequence on an X-ray transmission path of the X-ray source 31.

The imaging principle of the present disclosure is based on geometrical projection stripe mechanism of an X ray absorption grating. In essential, it follows principles of geometrical optics, instead of wave optics. As shown in Fig. 3, an incident X-ray is irradiated onto the beam splitting grating G1. A laterally periodic intensity distribution occurs in a geometrical projection region after the beam splitting grating G1. As the illumination X-ray has the spatial coherent length of only a few micrometers, much lower than the period (30 to 50 micrometers) of the grating G1, the diffraction effects become very weak and thus may be omitted. In a long distance after the beam splitting grating G1, the lateral distribution of the intensity becomes periodic. It may be known from calculation that when the period of the beam splitting grating G1 is 50 micrometers, the illumination X-ray will have a spatial length of 5 micrometers and the intensity distribution will still have good periodicity at a distance of 1 meter behind the beam splitting grating G1 and the period of the intensity distribution is equal to the period of the beam splitting grating G1. When an object 33 is placed behind the beam splitting grating G1, the object refracts the illumination X-ray to cause local distortion of the geometrical projection image of the beam splitting grating G1. Through the analyzer grating G2, the local distortion of the geometrical projection image is converted into intensity variation which may be recorded by the detector 32. The absorption, phase and scattering information may be obtained by analyzing the intensity variation caused by the object with respect to the intensity before the object is added.

In accordance with principles of geometrical optics, production of the projection image of the absorption grating G1 does not depend on effects such as diffraction. Thus, the embodiments of the present invention do not have any requirements for spatial coherence of the incident X-ray. Further, under polychromatic illumination, the geometrical projection image still occurs and has relatively high visibility, that is, the geometrical projection image is also not sensitive to the polychroism of the incident X-ray. Therefore, the X-ray source of the apparatus for the grating phase-contrast imaging according to the first embodiment of the present invention may use the conventional polychromatic X-ray source.

In accordance with the first embodiment of the present invention, the gratings G0, G1 and G2 are all absorption gratings. So-called absorption grating means that the illumination X-ray is absorbed completely by the scribe lines of the grating and the transmissivity of the grating is zero for the X-ray. The absorption grating may be made by producing a silicon substrate by lithographic process and then electroplating strong absorbing materials such as aurum or lead. In the embodiment, the absorption grating G2 may be replaced by structural scintillators.

As illustrated in Fig. 1, under illumination of spatial partially coherent X-ray, the diffraction intensity distribution of the X-ray will vary periodically in lateral direction at a special distance from the phase grating G1 and behind the phase grating G1. Such phenomenon is called as fraction Talbot self imaging effect and the distance is called as fraction Talbot distance. The spatial partially coherent illumination needs the period of the phase grating G1 is not greater than a spatial coherent length of the illumination X-ray.

In accordance with the first embodiment of the present invention, the gratings G1, G2 have periods of 30 to 50 micrometers and depth to width ratios not greater than 20. The apparatus for X-ray grating phase-contrast imaging according to embodiments of the present invention is based on geometrical projection stripe mechanism of an X ray absorption grating, that is, under illumination of the X-ray, the distribution of the intensity of the projection image of the grating G1 varies periodically in a direction perpendicular to an optical axis at a special distance from the beam splitting grating G1 and behind the beam splitting grating G1 (depending on period of grating). Such phenomenon is called as geometrical projection stripe mechanism. In the present application, the period of the beam splitting grating G1 is not limited by the spatial coherent length of the illumination X-ray, may be 30 to 50 micrometers. The refraction of the object 33 to the illumination X-ray may cause lateral local distortion of the geometrical projection image of the grating G1. Through the analyzer grating G2, such distortion is converted into local variation of the intensity which is recorded by the detector 32 arranged adjacent to and behind the grating G2. As shown in Table 1, with 80keV, absorptivity of 90% for the X-ray needs an absorption layer having a thickness of 545 micrometers. The corresponding absorption gratings G1, G2 have depth to width ratios not greater than 20, which may be achieved by the conventional micro-manufacturing technology. In contrast to it, in the conventional Talbot-Lau interferometer, the period of the grating cannot be greater than the spatial coherent length of the illumination X-ray and is typically lower than 5 micrometers. Thus, it causes the corresponding depth to width ratio of the grating to be greater than 100, which is not achievable by the conventional micro-manufacturing technology.

In accordance with the first embodiment of the present invention, the grating has a period of 30 to 50 micrometers. Thus, the conventional micro-manufacturing technology may be used to produce a large area (>100×100mm2) absorption grating, for example, an absorption grating of up to 200mm ×400mm used as the beam splitting grating G1 and the analyzer grating G2 of the embodiment of the present invention. Therefore, it may achieve large field-of-view for imaging.

In summary, by the conventional micro-manufacturing technology, the embodiment of the present invention may achieve X-ray phase-contrast imaging with large field-of-view (such as 200 mm×400 mm) and high energy (>60keV) and may be used in practice, such as clinical medicine.

Table 1 Requirements of various methods to depth to width ratio of the grating in high energy(80KeV) imaging

| X-ray energy of 80 keV | Talbot-Lau interferometer | Apparatus of the embodiment of the present invention |
|---|---|---|
| Thickness of absorption layer (90% absorptivity) | 545μm | 545μm |
| Period of grating | <5μm | 30~50μm |
| Depth to width ratio of grating | >100 | 5~20 |

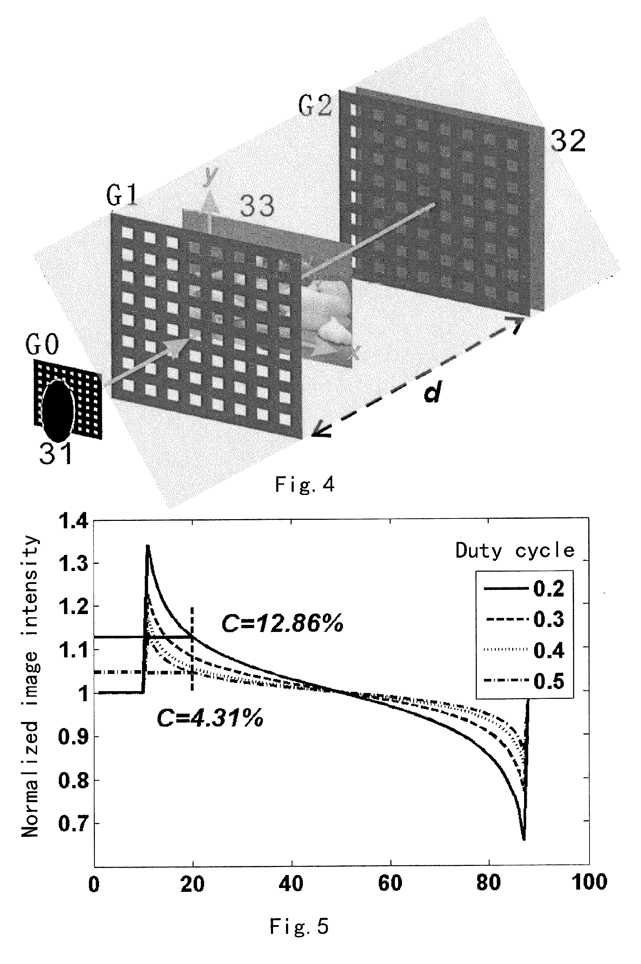

In accordance with the first embodiment of the present invention, the beam splitting grating G1 and the analyzer grating G2 have a duty cycle less than 0.5, preferably between 0.2 and 0.4. Through research, it is found that the imaging contrast of the object may be reduced when the period of the beam splitting grating G1 is 30 to 50 micrometers. In order to compensate for the reduction of the object image contrast (approximately in inverse proportion to period of the grating), the duty cycle of the grating G1 is optimized in design. Fig. 5 illustrates variation of the object image contrast as the duty cycle of the beam splitting grating G1. Taking a PMMA column in an air environment as an example, when the duty cycle of the grating G1 is 0.2, the image contrast is 12.86%. In the conventional method for the Talbot-Lau interferometer, the duty cycle of the grating G1 is 0.5 and the image contrast is only 4.31 %. That is, when the duty cycle of the grating G 1 is 0.2, the object image contrast is improved by over three times. Therefore, in the embodiment of the present invention, the object image contrast may be improved by optimizing the duty cycle of the beam splitting grating G 1.

In accordance with the first embodiment of the present invention, the distance between the beam splitting grating G1 and the analyzer grating G2 may be selected as required in practice. Depending on the specific requirements in practice, the distance d between the grating G1 and the grating G2 may be optimized in consideration of factors such as image contrast, exposure time or radiation dose, typically, the distance d may be 1 to 2 meters.

In the grating phase-contrast imaging, without considering effects of such as exposure time, the object image contrast is in direct proportion to the distance between the beam splitting grate G1 and the analyzer grating G2. In the conventional Talbot-Lau interferometer, the distance between the beam splitting grating G1 and the analyzer grating G2 should be equal to fraction Talbot distance of a certain order of the beam splitting grating G1, to achieve the highest image contrast. The fraction Talbot distance of the beam splitting grating G1 has only a few centimeters to more than ten but less than twenty centimeters. In accordance with the embodiment of the present invention, the distance between the beam splitting grating G1 and the analyzer grating G2 may be selected as required in practice, typically may be 1 to 2 meters, and thus the image contrast may be improved by 5 to 10 times.

In accordance with the first embodiment of the present invention, the gratings G0, G1, G3 are all curved surface gratings and meet normal incidence condition for an incident ray in an imaging field-of-view range all along. It may solve the problem that the edge portion of a plane grating transmits ray unevenly in conventional X-ray source cylindrical wave or spherical wave illumination. It may achieve large field-of-view for imaging (200mm × 400mm), and meets the requirements for clinical medical imaging, for example, glandula mammaria imaging.

In accordance with the first embodiment of the present invention, the detector 32 is used to record the variation of intensity. The detector may be of indirect detection type, for example charge coupled device (CCD), or may be of direct detection type, for example semiconductor detector.

When the apparatus for X-ray grating phase-contrast imaging of the embodiment of the present invention carries out an imaging operation, as shown in Fig.3, the object 33 is placed behind and adjacent to the beam splitting grating G1. The source grating G0 divides a conventional X-ray source into a plurality of separate slit sources. The beam splitting grating G1 produces an intensity array in a plane in which the analyzer grating G2 is located. The refraction of the object 33 to the X-ray causes lateral movement of the intensity array which is detected by the analyzer grating G2 and converted into intensity variation that is recordable by the detector.

**Second Embodiment**

The second embodiment of the present invention involves the same apparatus and method for X-ray grating phase-contrast imaging as those in the first embodiment of the present invention except that the radiation dose of the X-ray source 31 of the phase-contrast imaging apparatus in the second embodiment of the present invention is 20% of the dose in the conventional absorption contrast imaging, i.e., the radiation dose received by the object is 10% to 20% of the dose in the conventional absorption contrast imaging, so as to achieve an image with the same signal to noise ratio as that of the image in the conventional absorption contrast imaging.

The radiation dose received by the object is associated with the signal to noise ratio of the imaging method directly. In comparison with the conventional absorption contrast imaging method, the hard X-ray phase-contrast imaging method has one of advantages, i.e., improving the signal to noise ratio of the object imaging. The signal to noise ratio of the object image meets:

$$SNR \propto \sqrt{N} * C$$

where SNR is a signal to noise ratio, N is number of photons of the illumination X-ray at the plane in which the object is located, C is an image contrast of the sample. In the hard X-ray waveband, for primary constituent elements of human tissues such as carbon, hydrogen, oxygen, nitrogen, the refractivity phase term is greater than the absorption term by at least two orders of magnitude. Thus, the phase-contrast for the hard X-ray is much greater than the absorption contrast. From the known experiment results, it is estimated conservatively that the phase-contrast is five times of the absorption contrast, that is, $C_{\text{phase}}=5*C_{\text{absorption}}$.

When the signal to noise ratio of the phase-contrast imaging method is equal to the signal to noise ratio of the absorption contrast imaging, $SNR_{\text{phase}}=SNR_{\text{absorption}}$, then

$$\frac{N_{\text{phase}}}{N_{\text{absorption}}} = \left(\frac{C_{\text{absorption}}}{C_{\text{phase}}}\right)^2 = \frac{1}{25}$$

In the method for X-ray phase-contrast imaging with high contrast and low dose, the object is placed behind the beam splitting grating G1. Thus, only the loss of number of photons caused by the absorption of the analyzer grating G2 is necessary to be considered. Assuming that the grating G2 has a duty cycle of 0.2, the equivalent number of photons meets:

$$\frac{N_{\mathrm{phase}}}{N_{\mathrm{absorption}}} = \frac{1}{25 * 0.2} = 0.2$$

As the radiation dose received by the object is in direct proportion to the equivalent number of photons, the radiation dose of the phase-contrast imaging will be 20% of the conventional absorption contrast imaging, in consideration of the same signal to noise ratio of image.

**Third Embodiment**

Fig. 4 shows the third embodiment of the present invention. The apparatus and method for X-ray grating phase-contrast imaging provided by the embodiment of the present invention are not only applicable for one-dimensional circumstance, but also may be applied in two-dimensional circumstance. As shown in Fig. 4, in view of the first embodiment, the beam splitting grating G1 and the analyzer grating G2 are both two-dimensional absorption gratings. The beam splitting grating G1 and the analyzer grating G2 may be made by direct production, or by combining one-dimensional absorption gratings.

Similar to the one-dimensional circumstance, the object is placed behind and adjacent to the beam splitting grating G1 to reduce the radiation dose. The object refracts and scatters the X-ray to cause disturbances of the two-dimensional intensity array in x and y directions. By means of analyzing these disturbances, a plurality of images of the object such as absorption, refraction in x direction, refraction in y direction, scattering in x direction, and scattering in y direction can be extracted, so as to characterize the object more completely and accurately.

The experiment processes and data will be provided below.

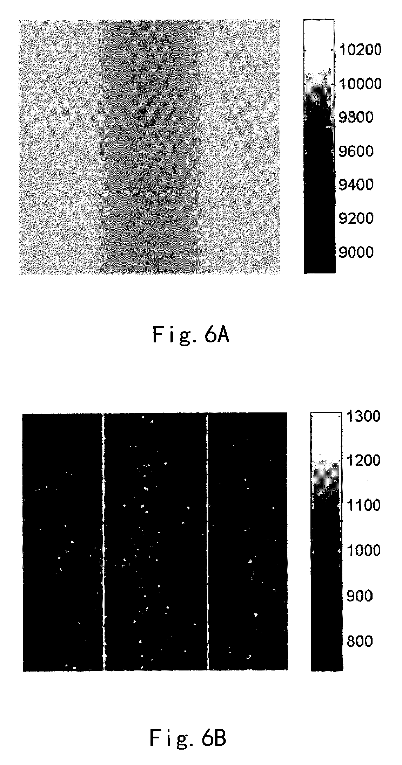

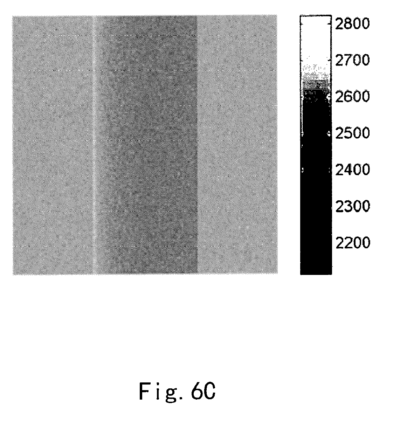

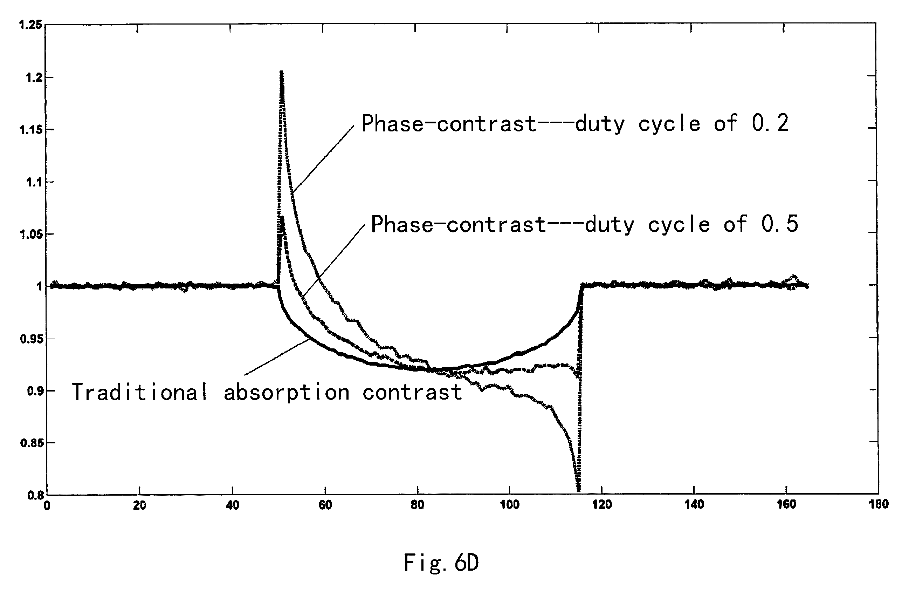

1) In experiments, the lateral displacement relative to each other of the beam splitting grating G1 and the analyzer grating G2 is kept constantly as a quarter of period of the analyzer grating. At that time, the object image contrast recorded by the imaging apparatus becomes maximum and the required radiation dose becomes minimum. In order to prove the feasibility of the proposed imaging apparatus and method, a series of comparison experiments are carried out. A column of Polymethy Methacrylate (PMMA) with a diameter of 4 millimeters in an air environment is selected as the sample. The X-ray has equivalent energy of 80keV. The period of the grating is 30 micrometers. The axial distance between the grating G1 and the grating G2 is two meters. The conventional absorption contrast imaging, the contrast imaging with grating duty cycle of 0.2 and the contrast imaging with grating duty cycle of 0.5 were carried out on a sample respectively. The results are shown in Fig.6A, Fig.6B and Fig.6C, respectively. In order to quantify the image contrast for different imaging methods and compare them with each other, Fig. 6D shows comparisons of cross sectional profiles shown in Figs. 6A to 6C. In order to remove the effects of statistic noises in images, the cross sectional profiles are averaged axially and are normalized on the basis of the background. As illustrated in Fig. 6D, when the grating duty cycle is 0.2, the image contrast is 20.06%. In the conventional Talbot-Lau interferometer method, the grating duty cycle is 0.5, and the image contrast is only 7.83%. In the traditional absorption contrast imaging, the image contrast is only 4.30%. That is, when the grating duty cycle is 0.2, the image contrast of the object is improved by 4.67 times compared with the traditional absorption contrast image and improved by 2.56 times compared with the conventional Talbot-Lau interferometer method. Therefore, one of characteristics of the present application is to enhance the image contrast of object and reduce the radiation dose by optimizing the duty cycle of the grating.

2) The original images collected in the experiments not only contain phase information of the object, but also contain absorption information and scattering information of the object. In practice, Computerized Tomography (CT) Reconstruction and quantification analysis and the like need pure phase information of the object as an input. Thus, it is necessary to separate the phase information of the object from the original image. In the experimental equipment proposed by the present application, a plurality of images are collected at various relative positions of the grating by scanning the grating laterally to achieve the absorption, phase information of the object from the scattering information of the object.

A column of Polymethy Methacrylate (PMMA) with a diameter of 5 millimeters and a column of polyformaldehyde (POM) with a diameter of 10 millimeters in an air environment are selected as samples. The period of the grating is 50 micrometers. The duty cycle of the grating is 0.33. The distance between the grating G1 and the grating G2 is 1 meter.

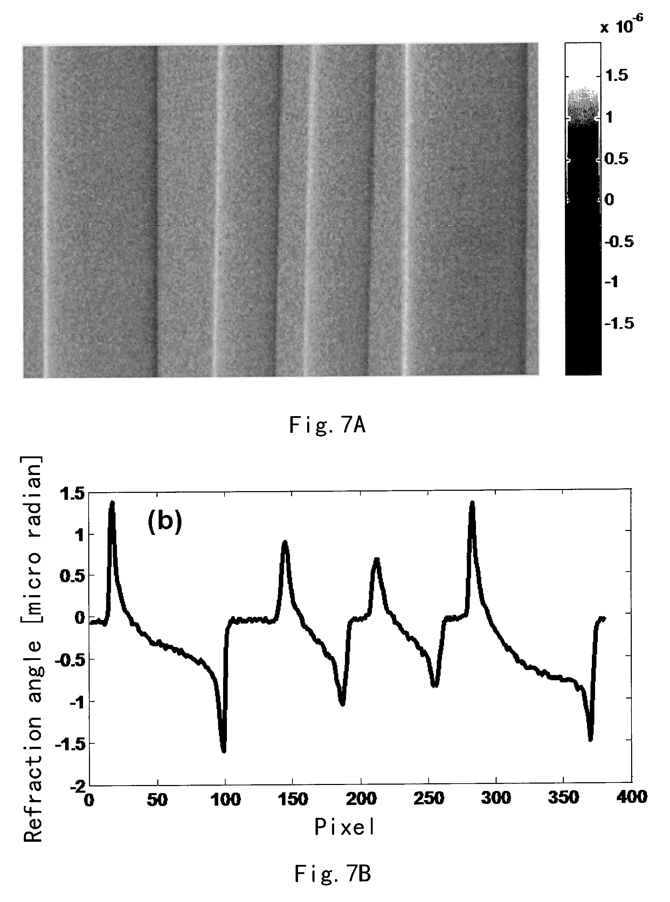

The grating G2 is scanned laterally and an image is recorded at each scan point. The plurality of images recorded are calculated to obtain the refraction images of the column of PMMA and the column of POM, as shown in Fig. 7(a). In order to explain the quantification of the imaging apparatus according to an embodiment of the present invention, Fig. 7(b) shows cross sectional profiles of the refraction image of the sample. For the column of PMMA with a diameter of 10 millimeters, the refraction angle has a theoretical maximum value of 1.52 micro-radian and a measured value of 1.49 micro-radian in experiments. The measured value is in good coincidence with theoretical value, which proves the imaging apparatus proposed in the embodiment of the present invention can acquire the refraction information of the object quantitatively.

On the basis of the quantified extraction of the refraction angle of the object, in combination with the algorithm of Computerized Tomography (CT) reconstruction, the three-dimensional spatial distribution of real part of the refractivity of the object can be obtained:

$$\delta(x,y,z) = -\int_0^\pi d\Theta \int_{-\infty}^\infty \left[ \theta_r(x_r, \Theta, z) * F^{-1}\left(\frac{|\rho|}{2\pi j\rho}\right)\right] \cdot \delta(x\cos\Theta + y\sin\Theta - x_r)dx_r$$

wherein $\delta$ is the real part of the refractivity of the object, $\theta_r(x_r, \Theta, z)$ is a refraction angle extracted under a projection angle $\Theta$, $\rho$ is a spatial frequency, $F^{-1}$ is inverse Fourier transformation. The above paragraphs are explained with reference to three-dimensional tomography reconstruction with illumination of parallel beams. For more generic illumination of fan beam, the three-dimensional tomography reconstruction may be achieved by a variable substitution method.

The above specific embodiments are intended to explain the objects, solutions and advantages of the present application in detail. It should be noted that the above embodiments are provided only by way of examples, other than limiting the present invention. All changes, alternatives or modifications which are made within the principles and spirit of the present application should fall within the scopes of the present invention.

**Claims**

1. An apparatus for X-ray grating phase-contrast imaging, comprising:

    a source emitter (31), a source grating (G0), a beam splitting grating (G1), an analyzer grating (G2) and a detector (32) arranged in sequence on a transmission path of the source emitter (31), wherein the beam splitting grating (G1) has a period of 30 to 50μm and a depth to width ratio not greater than 20.

2. The apparatus for X-ray grating phase-contrast imaging according to claim 1, wherein the source emitter (31) is a hard X-ray source.

3. The apparatus for X-ray grating phase-contrast imaging according to claim 2, wherein the hard X-ray source has energy greater than 60keV.

4. The apparatus for X-ray grating phase-contrast imaging according to claim 1, wherein the source grating (G0), the beam splitting grating (G1) and the analyzer grating (G2) are all absorption gratings.

5. The apparatus for X-ray grating phase-contrast imaging according to claim 1, wherein the beam splitting grating (G1) has a duty cycle less than 0.5.

6. The apparatus for X-ray grating phase-contrast imaging according to claim 1, wherein the beam splitting grating (G1) has a size greater than 100mm×100mm.

7. The apparatus for X-ray grating phase-contrast imaging according to claim 1, wherein there is a distance of 1 to 2 meters between the beam splitting grating (G1) and the analyzer grating (G2).

8. The apparatus for X-ray grating phase-contrast imaging according to claim 1, wherein the source grating, the beam splitting grating and the analyzer grating (G0, G1, G3) are all curved surface gratings and meet normal incidence condition for an incident ray in an imaging field-of-view range all along.

9. The apparatus for X-ray grating phase-contrast imaging according to claim 1, wherein the beam splitting grating

(G1) and the analyzer grating (G2) are both two dimensional gratings.

10. The apparatus for X-ray grating phase-contrast imaging according to claim 1, wherein the source emitter (31) is a neutron emitter.

11. A method for X-ray grating phase-contrast imaging, comprising:

arranging a source grating (G0), a beam splitting grating (G1), an analyzer grating (G2) and a detector (32) in sequence on a transmission path of a source emitter (31);
putting an object (33) against the beam splitting grating (G1) and towards the analyzer grating (G2);
splitting a beam source from the source emitter (31) into a plurality of separate beam sources by the source grating (G0) and generating an intensity array in a plane in which the analyzer grating (G2) is located by the beam splitting grating (G1), wherein the object (33) refracts a ray from the beam source to cause a lateral movement of the intensity array, which is detected by the analyzer grating (G2) and converted into an intensity variation which is recordable by the detector (32), and
wherein the beam splitting grating (G1) has a period of 30 to 50 $\mu$m and a depth to width ratio not greater than 20.

12. The method for X-ray grating phase-contrast imaging according to claim 11, wherein the source emitter (31) is a hard X-ray source.

13. The method for X-ray grating phase-contrast imaging according to claim 12, wherein the hard X-ray source has energy greater than 60keV.

14. The method for X-ray grating phase-contrast imaging according to claim 11, wherein the source grating (G0), the beam splitting grating (G1) and the analyzer grating (G2) are all absorption gratings.

15. The method for X-ray grating phase-contrast imaging according to claim 11, wherein the beam splitting grating (G1) has a duty cycle less than 0.5.

16. The method for X-ray grating phase-contrast imaging according to claim 11, wherein the beam splitting grating (G1) has a duty cycle between 0.2 and 0.4.

17. The method for X-ray grating phase-contrast imaging according to claim 11, wherein the beam splitting grating (G1) has a size greater than 100mm×100mm.

18. The method for X-ray grating phase-contrast imaging according to claim 11, wherein there is a distance of 1 to 2 meters between the beam splitting grating (G1) and the analyzer grating (G2).

19. The method for X-ray grating phase-contrast imaging according to claim 11, wherein the source grating, the beam splitting grating and the analyzer grating (G0, G1, G3) are all curved surface gratings and meet normal incidence condition for the incident ray in an imaging field-of-view range all along.

20. The method for X-ray grating phase-contrast imaging according to claim 11, wherein the beam splitting grating (G1) and the analyzer grating (G2) are both two dimensional gratings.

21. The method for X-ray grating phase-contrast imaging according to claim 1, wherein the source emitter (31) is a neutron emitter.

**G1**

$d = 0$          $d = p^2/\lambda$          $d = 2p^2/\lambda$

Fig. 1

$L$          $Dn$

**21**          **23**          **22**

G0          G1          G2

Fig. 2

Fig. 3

Fig. 4

Fig. 5

Fig. 6A

Fig. 6B

Fig. 6C

Fig. 6D

Fig. 7A

Fig. 7B

| INTERNATIONAL SEARCH REPORT | International application No. |
| --- | --- |
| | **PCT/CN2014/071096** |

**A. CLASSIFICATION OF SUBJECT MATTER**

A61B 6/02 (2006.01) i; G01N 23/04 (2006.01) i

According to International Patent Classification (IPC) or to both national classification and IPC

**B. FIELDS SEARCHED**

Minimum documentation searched (classification system followed by classification symbols)

A61B, G01N

Documentation searched other than minimum documentation to the extent that such documents are included in the fields searched

Electronic data base consulted during the international search (name of data base and, where practicable, search terms used)

CPRSABS, CNTXT, CNKI, VEN: phase contrast, optical grating, x w ray, phase?, contrast, grating?, period+, aspect w ratio

**C. DOCUMENTS CONSIDERED TO BE RELEVANT**

| Category* | Citation of document, with indication, where appropriate, of the relevant passages | Relevant to claim No. |
| --- | --- | --- |
| Y | EP 2060909 A1 (SUISSE ELECTRONIQUE MICROTECH), 20 May 2009 (20.05.2009), description, paragraphs [0036]-[0100], and figures 1-12 | 1-21 |
| Y | CN 101532969 A (NUCTECH COMPANY LIMITED et al.), 16 September 2009 (16.09.2009), description, page 1, paragraph 2 to page 16, paragraph 5, and figures 1-11 | 1-21 |
| Y | EP 1447046 A1 (SCHERRER INST PAUL), 18 August 2004 (18.08.2004), description, paragraphs [0036]-[0046], and figures 1 and 2 | 1-21 |
| Y | CN 102802529 A (ROYAL DUTCH PHILIPS ELECTRONICS LTD.), 28 November 2012 (28.11.2012), description, paragraphs [0016]-[0074], and figures 1-5c | 9, 10, 20, 21 |
| A | CN 101257851 A (PAUL SCHERRER INSTITUTE), 03 September 2008 (03.09.2008), the whole document | 1-21 |
| A | CN 102655809 A (ROYAL DUTCH PHILIPS ELECTRONICS LTD.), 05 September 2012 (05.09.2012), the whole document | 1-21 |

☒ Further documents are listed in the continuation of Box C.        ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 07 August 2014 (07.08.2014) | **18 August 2014 (18.08.2014)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **ZHANG, Qingnan** Telephone No.: (86-10) **62085610** |
| --- | --- |

Form PCT/ISA/210 (second sheet) (July 2009)

## INTERNATIONAL SEARCH REPORT

International application No.

**PCT/CN2014/071096**

**C (Continuation).** DOCUMENTS CONSIDERED TO BE RELEVANT

| Category* | Citation of document, with indication, where appropriate, of the relevant passages | Relevant to claim No. |
|---|---|---|
| A | US 2011243300 A1 (FUJIFILM CORP.), 06 October 2011 (06.10.2011), the whole document | 1-21 |

Form PCT/ISA/210 (continuation of second sheet) (July 2009)

## INTERNATIONAL SEARCH REPORT
### Information on patent family members

International application No.

**PCT/CN2014/071096**

| Patent Documents referred in the Report | Publication Date | Patent Family | Publication Date |
|---|---|---|---|
| EP 2060909 A1 | 20 May 2009 | EP 2060909 B1 | 07 September 2011 |
| | | US 7924973 B2 | 12 April 2011 |
| | | US 2009128830 A1 | 21 May 2009 |
| | | JP 2009150875 A | 09 July 2009 |
| | | AT 524056 T | 15 September 2011 |
| CN 101532969 A | 16 September 2009 | CN 101576515 A | 11 November 2009 |
| | | CN 101576515 B | 04 July 2012 |
| | | CN 101532969 B | 17 April 2013 |
| | | HK 1137510 A1 | 11 October 2013 |
| EP 1447046 A1 | 18 August 2004 | AU 2003275964 A1 | 06 September 2004 |
| | | WO 2004071298 A1 | 26 August 2004 |
| CN 102802529 A | 28 November 2012 | US 2012099702 A1 | 26 April 2012 |
| | | EP 2442722 A1 | 25 April 2012 |
| | | WO 2010146503 A1 | 23 December 2010 |
| CN 101257851 A | 03 September 2008 | AU 2006257026 A1 | 14 December 2006 |
| | | WO 2006131235 A1 | 14 December 2006 |
| | | AU 2006257026 B2 | 04 March 2010 |
| | | JP 5162453 B2 | 13 March 2013 |
| | | EP 1887936 A1 | 20 February 2008 |
| | | CA 2610934 A1 | 14 December 2006 |
| | | CN 101257851 B | 15 June 2011 |
| | | JP 2008545981 A | 18 December 2008 |
| | | EP 1731099 A1 | 13 December 2006 |
| | | US 7889838 B2 | 15 February 2011 |
| | | EP 1887936 B1 | 25 December 2013 |
| | | US 2009092227 A1 | 09 April 2009 |
| CN 102655809 A | 05 September 2012 | RU 2012128794 A | 20 January 2014 |
| | | EP 2509503 A1 | 17 October 2012 |
| | | US 2012307966 A1 | 06 December 2012 |
| | | WO 2011070493 A1 | 16 June 2011 |
| | | JP 2013513414 A | 22 April 2013 |
| US 2011243300 A1 | 06 October 2011 | JP 2011227041 A | 10 November 2011 |
| | | US 8755487 B2 | 17 June 2014 |

Form PCT/ISA/210 (patent family annex) (July 2009)

**EP 3 069 659 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MOMOSE A ; FUKUDA J.** Phase contrast radiographs of nonstained rat cerebellar specimen. *Med. Phys.,* 1995, vol. 22, 375
- **BONSE U ; HART M.** An X-ray interferometer. *Appl. Phys. Lett.,* 1965, vol. 6, 155
- **MOMOSE A ; TAKEDA T ; ITAI Y.** Phase-contrast X-ray computed tomography for observing biological soft tissues. *Nat. Med.,* 1996, vol. 2, 473
- **MOMOSE A ; KAWAMOTO S ; KOYAMA I ; HAMAISHI Y ; TAKAI K ; SUZUKI Y.** Demonstration of X-ray Talbot interferometry. *Jpn. J. Appl. Phys.,* 2003, vol. 42 (7B), L866
- **PFEIFFER F ; WEITKAMP T ; BUNK O et al.** Phase retrieval and differential phase-contrast imaging with low-brilliance X-ray sources. *Nat. Phys.,* 2006, vol. 2, 258
- **DAVIS T J ; GAO D ; GUREYEV T E et al.** Phase-contrast imaging of weakly absorbing materials using hard X-rays. *Nature,* 1995, vol. 373, 595
- **CHAPMAN D ; THOMLINSON W ; JOHNSTON RE et al.** Diffraction enhanced x-ray imaging. *Phys. Med. Biol.,* 1997, vol. 42, 2015
- **SNIGIREV A ; SNIGIREVA I ; KOHN V ; KUZNETSOV S ; SCHELOKOV I et al.** On the possibilities of x-ray phase contrast microimaging by coherent high-energy synchrotron radiation. *Rev. Sci. Instrum.,* 1995, vol. 66, 5486
- **WILKINS S W ; GUREYEV T E ; GAO D et al.** Phase-contrast imaging using polychromatic hard X-rays. *Nature,* 1996, vol. 384, 335